(19) Europäisches Patentamt
European Patent Office
Office européen des brevets

(11) Publication number: 0 085 514
A2

# (12) EUROPEAN PATENT APPLICATION

(21) Application number: 83300306.4

(22) Date of filing: 21.01.83

(51) Int. Cl.[3]: A 61 K 45/06
A 61 K 31/135, A 61 K 31/18
A 61 K 31/165
//(A61K31/18, 31/135),
(A61K31/165, 31/135)

(30) Priority: 28.01.82 GB 8202449

(43) Date of publication of application:
10.08.83 Bulletin 83/32

(84) Designated Contracting States:
CH DE FR GB IT LI NL

(71) Applicant: BEECHAM GROUP PLC
Beecham House Great West Road
Brentford Middlesex(GB)

(72) Inventor: Arch, Johathon Robert Sanders
33 Chapel Way
Epsom Surrey(GB)

(74) Representative: Russell, Brian John et al,
European Patent Attorney Beecham Pharmaceuticals
Great Burgh Yew Tree Bottom Road
Epsom Surrey, KT18 5XQ(GB)

## (54) Compositions comprising a beta-antagonist and a beta-agonist, their preparation, and their use in treating obese humans and animals.

(57) A pharmaceutically acceptable composition comprising a β-agonist and a β-antagonist selected from propranolol, sotalol, atenolol, metoprolol, and salts or esters thereof, useful in the treatment of obese humans or animals. The composition is particularly useful in the treatment of an obese human, or animal suffering from a heart complaint. In which case the treatment with a β-agonist alone may unacceptably aggravate the heart complaint.

EP 0 085 514 A2

Croydon Printing Company Ltd.

# Compositions comprising a β-antagonist and a β-agonist, their preparation, and their use in treating obese humans and animals

This invention relates to a pharmaceutical composition having anti-obesity activity coupled with low cardiac stimulation activity.

Our European Published Patent Application No. 0006735 discloses compounds of the formula (I):

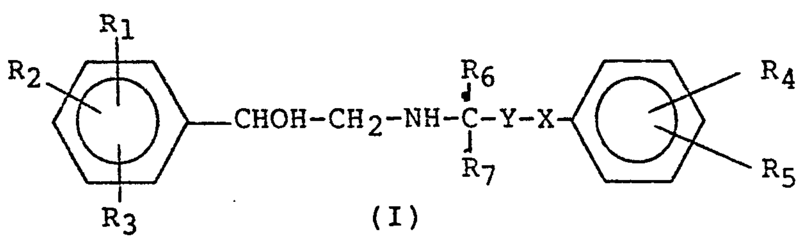

(I)

or pharmaceutically acceptable salts thereof, wherein $R_1$ is a hydrogen, fluorine or chlorine atom or a hydroxyl, hydroxymethyl, methyl, methoxyl, amino, formamido, acetamido, methylsulphonylamido, nitro, benzyloxy, methylsulphonylmethyl, ureido, trifluoromethyl or p-methoxybenzylamino group; $R_2$ is a hydrogen, fluorine or chlorine atom or a hydroxyl group; $R_3$ is a hydrogen or chlorine atom or a hydroxyl group; $R_4$ is a carboxylic acid group or a salt, ester or amide thereof; $R_5$ is a hydrogen, chlorine or fluorine atom or a methyl, methoxyl or hydroxyl group or a carboxylic acid group or a salt, ester or amide thereof; $R_6$ is a hydrogen atom or a methyl, ethyl or

propyl group; $R_7$ is a hydrogen atom or a methyl, ethyl or propyl group; X is an oxygen atom or a bond; and Y is an alkylene group of up to 6 carbon atoms or a bond.

The compounds of formula (I) are β-adrenoceptor agonists having anti-obesity properties. The principal mechanism of the anti-obesity action of these compounds, as with other β-agonists, is to increase the energy expenditure in animals rather than decrease energy intake.

A disadvantage of some β-agonists is that they tend to increase the heart rate of the animal, as well as increase the energy expenditure, and much research has been carried out to find β-agonists which can increase the energy expenditure without appreciably increasing the heart rate.

We have now surprisingly found that, by administering to an animal a β-agonist together with a selected β-antagonist, it is possible to decrease significantly the cardiac effect of the β-agonist without appreciably affecting the energy expenditure. On theoretical grounds, it would be expected that a β-antagonist would decrease both the cardiac effect and energy expenditure effect of a β-agonist. The discovery that it decreases the cardiac effect but not the energy expenditure effect is contrary to what would have been predicted from theory.

Accordingly, the invention provides a pharmaceutical composition comprising a β-agonist and a β-antagonist selected from propranolol, sotalol, atenolol and metoprolol and salts or esters thereof, together with a pharmaceutically acceptable carrier or excipient.

The term β-agonist as used herein is intended to include a compound which is capable of increasing the activity of the endogenous β-agonists noradrenaline and adrenaline. A preferred example of such a compound is ephedrine or an acid addition salt of ephedrine.

Preferred β-agonists are those disclosed in our above mentioned European Patent Application No. 0006735.

Other suitable β-agonists are those disclosed in our U.S. Patent Nos. 4,338,333, 4,309,443, 4,329,358, 4,341,793, our Published European Patent Application Nos. 0,021,636, 0,040,000, 0,040,915, 0,051,917, 0,052,963, 0,066,351, 0,061,907, 0,063,004, our European Patent Application Nos. 82302930.1, 82303507.6, and our U.K. Patent Application Nos. 8207325, 8212355, 8220662, 8228753 and 8224110.

Further suitable β-agonists are salbutamol and fenoterol and salts thereof.

The proportions of the active ingredients in the compositions of the invention can vary widely.

Preferably, the weight ratios of β-agonist to β-antagonist can be from about 1:100 to 10:1.

The compositions of the present invention can be made by conventional techniques in the art, usually by simple admixture of the active ingredients with the carrier or excipient.

Usually the compositions of the present invention will be adapted for oral administration although compositions for administration by other routes, such as by injection are also envisaged.

Suitable compositions for oral administration are unit dosage forms such as tablets and capsules. Other fixed-unit dosage forms, such as powders presented in sachets, may also be suitable.

In accordance with conventional pharmaceutical practice the carrier may comprise a diluent, binder, filler, disintegrant, wetting agent, lubricant, dye, and flavouring agent.

Typical carriers may comprise microcrystalline cellulose, starch, sodium starch glycollate, polyvinylpyrrolidone, polyvinylpolypyrrolidone, magnesium stearate, sodium lauryl sulphate and sucrose.

Most suitably the composition will be provided in unit dose form. Such unit doses will normally comprise from 1 to 500 mg of the β-antagonist with from 0.1 to 500 mg of the β-agonist, suitably from 2 to 400 mg of the β-antagonist with from 0.5 to 400 mg of the β-agonist and favourably from 4 to 250 mg of the β-antagonist with from 1 to 200 mg of the β-agonist.

The present invention also provides a method for treating obesity in humans or animals, which method comprises administering an effective, non-toxic amount of a composition of the invention to obese humans or animals.

In treating obese humans, the composition may be taken in doses, such as those described above, one to six times a day in a manner such that the total daily dose for a 70 kg adult will generally be about from 1 to 1500 mg of the β-antagonist with from 1 to 1000 mg of the β-agonist and more usually from 10 to 1000 mg of the β-antagonist with from 1 to 600 mg of the β-agonist.

In treating obese animals, especially dogs, the composition may be administered by mouth, usually once or twice a day and at about 0.1 to 20 mg/kg of the β-antagonist with from 0.01 to 10 mg/kg of the β-agonist, for example from 1 to 10 mg/kg of the β-antagonist with from 0.1 to 2 mg/kg of the β-agonist.

The invention will now be illustrated with reference to the following examples.

EXAMPLES

The following ingredients may be mixed together and filled into a two part bond gelatine capsule:

| | |
|---|---|
| Propranolol hydrochloride | 50 mg |
| 1R,2'R-N(2-[4-Carbomethoxyphenyl]-1-methylethyl)-2'-(3-chlorophenyl)-2'-hydroxyethanamine fumarate | 30 mg |
| Lactose | 150 mg |

This formulation is suitable for administration to humans per os.

In a similar fashion, the following combinations of active ingredients may be used:

| β-antagonist | | β-agonist | |
|---|---|---|---|
| Propranolol hydrochloride | 50 mg | Salbutamol sulphate | 4 mg |
| Propranolol hydrochloride | 50 mg | Salbutamol sulphate | 8 mg |
| Propranolol hydrochloride | 50 mg | Fenoterol hydrobromide | 10 mg |
| Propranolol hydrochloride | 30 mg | l-Ephedrine hydrochloride | 80 mg |

| | | | |
|---|---|---|---|
| Sotalol hydrochloride | 100 mg | N-[2-(4-Hydroxy-methylphenyl)-1-methylethyl]-2-(2-fluorophenyl)-2-hydroxy ethanamine fumarate | 30 mg |
| Atenolol | 100 mg | N-[1-R-2-(4-Methyl-phenyl)-1-methylethyl]-2-R-2-hydroxy-2-phenyl ethanamine | 75 mg |
| Propranolol hydrochloride | 50 mg | N-[2-(4-carbomethoxy-methoxyphenyl)-1-methylethyl]-2-hydroxy-2-(3-trifluoromethyl-phenyl)ethanamine | 10 mg |

BIOLOGICAL DATA

(i) Energy Expenditure

Male Sprague-Dawley rats (body weight 150-200 g) are fasted overnight before being dosed with a solution or suspension of the compounds in water. The β-agonist and antagonist are dosed within 5 mins of each other.

Each rat is placed in a box and room air, at 23°C, is drawn through the box at about 45 litres/hour. The percentage oxygen in room air and air leaving each box is measured every 10 to 15 minutes. Energy expenditure is calculated from the difference between the percentage oxygen in room air and air leaving the box, and from the rate of air flow, using equations similar to those presented by J.B. De V. Weir in The Journal of Physiology 109, 1-9 (1949). Rates of energy expenditure are shown in the Table for the period 0 to 3 hours after dosing and are expressed as percentages of the mean rate of energy expenditure by three rats that were dosed with water.

A different method is used for experiments that involve l-ephedrine. The rats are not fasted overnight. They are anaesthetised with sodium pentobarbitone (60 mg/kg i.p.) before being placed in a closed-circuit respirometer at 29°C. The respirometer contains soda lime and silica gel to absorb carbon dioxide and water so that oxygen consumption can be measured as the volume of oxygen that must be reintroduced to correct for any fall in pressure in the chamber. Rates of energy expenditure shown in the Table are rates of oxygen consumption for the period 20 to 30 minutes after dosing expressed as percentages of the mean rate of energy expenditure of four rats that were dosed with saline.

(ii) Heart Rate

The pressure pulse from the rat's tail blood flow is recorded via a sensor round the tail and displayed on a storage oscilloscope. Heart rate is obtained by counting the number of pulses over a fixed time interval (5 seconds). To ensure adequate peripheral blood flow, the rats are kept in an incubator at 35°C for 38 minutes before each recording. After an initial reading, the rats are dosed orally, then further readings taken every 45 minutes. Results shown in the Table are expressed as the mean net change in heart rate of drug-treated groups after subtracting changes in a control group.

In oxygen consumption tests that involve ephedrine the heart rate is measured 33 minutes after dosing. A cardiac puncture needle is positioned with its tip lying in the left ventricle. The needle is connected to a pressure transducer. The heart rate is recorded 2 to 3 minutes after cardiac puncture when the rate is stable.

TABLE

| AGONIST | DOSE (mg/kg) and route | ANTAGONIST | DOSE (mg/kg) and route | * MAXIMUM INCREASE IN HEART RATE (beats per min) | % OF CONTROL ENERGY EXPENDITURE 0-3h* |
|---|---|---|---|---|---|
| Salbutamol sulphate | 50 po | - | - | 112 | 145 |
| | 50 po | Propranolol | 20 po | 58 | 137 |
| Salbutamol sulphate | 50 po | - | - | 152 | 151 |
| | 50 po | Sotalol | 25 po | 65 | 143 |
| Fenoterol hydrobromide | 30 po | - | - | 107 | 141 |
| | 30 po | Propranolol | 10 po | 49 | 137 |
| 1-Ephedrine hydrochloride | 20 ip | - | - | 125 | 155 |
| | 20 ip | Propranolol | 1 ip | 64 | 163 |
| | 20 ip | Propranolol | 3 ip | 20 | 141 |
| 1R,2'R-N-[2-(4-Carbomethoxyphenyl)-1-methylethyl]-2'-(3-chlorophenyl)-2'-hydroxyethanamine fumarate | 5.6 po | - | - | 102 | 144 |
| | 5.6 po | Propranolol | 10 po | 15 | 138 |
| N-[2-(4-Hydroxymethylphenyl)-1-methylethyl]-2-(2-fluorophenyl)-2-hydroxyethanamine fumarate | 3 po | - | - | 77 | 131 |
| | 3 po | Sotalol | 10 po | -5 | 120 |
| N-[1-R-2-(4-Methylphenyl)-1-methylethyl]-2-R-2-hydroxy-2-phenylethanamine | 7.5 po | - | - | 135 | 144 |
| | 7.5 po | Atenolol | 10 po | 48 | 144 |
| N-[2-(4-Carbomethoxymethoxyphenyl)-1-methylethyl]-2-hydroxy-2-(3-trifluoromethylphenyl)ethanamine | 2.1 po | - | - | 70 | 156 |
| | 2.1 po | Propranolol | 10 po | 28 | 155 |

* Approximately 30 mins after dosing for experiments involving ephedrine.

Where negative increases in heart rate are shown, this is the decrease in heart rate at the time when the agonist had its maximum effect.

The above results clearly demonstrate that the heart rate is reduced by the addition of a β-antagonist, without there being any substantial effect on the energy expenditure.

CLAIMS

1. A pharmaceutically acceptable composition having anti-obesity activity, characterised in that it comprises a β-agonist and a β-antagonist selected from propranolol, sotalol, atenolol, metoprolol, and salts or esters thereof.

2. A composition as claimed in claim 1, characterised in that the β-agonist is selected from 1R,2'R-N(2-[4-carbomethoxyphenyl]-1-methylethyl)-2'-(3-chlorophenyl)-2'-hydroxyethanamine, N-[2-(4-hydroxymethylphenyl]-2-(2-fluorophenyl)-2-hydroxyethanamine, N-[1-R-2-(4-methylphenyl)-1-methylethyl]-2-R-2-hydroxy-2-phenylethanamine, N-[2-(4-carbomethoxymethoxyphenyl)-1-methylethyl]-2-hydroxy-2-(3-trifluoromethylphenyl)ethanamine, and acid addition salts thereof.

3. A composition as claimed in claim~~s~~ 1 ~~or 2,~~ characterised in that the β-agonist is salbutamol, fenoterol, 1-ephedrine, and salts thereof.

4. A composition as claimed in claims 1 to 3, characterised in that the range of weight ratio~~s~~ of β-agonist to β-antagonist is from 1:100 to 10:1.

5. A composition as claimed in claims 1 to 4, in unit dosage form.

6. A composition as claimed in claim 5, characterised in that the unit dose comprises from 1 to 500 mg of β-antagonist and from 0.1 to 500 mg of β-agonist.